# FASCICULE DE BREVET EUROPEEN

(11) **EP 0 751 740 B1**
(45) Date de publication et mention de la délivrance du brevet: **10.06.1998**
(21) Numéro de dépôt: 95914391.8
(22) Date de dépôt: 21.03.1995
(51) Int. Cl.: A61B 5/00

(54) **DISPOSITIF D'IMAGERIE ENDOSCOPIQUE OU FIBROSCOPIQUE EN FLUORESCENCE DANS L'INFRAROUGE**
BILDFORMUNGSANORDNUNG MITTELS INFRAROT-FLUOREZENZ FÜR EIN ENDOSKOP ODER FIBERSKOP
ENDOSCOPIC OR FIBEROPTIC IMAGING DEVICE USING INFRARED FLUORESCENCE

(30) Priorité: 21.03.1994 FR 9403296
(43) Date de publication de la demande: 08.01.1997
(73) Titulaire: Societe d'Etudes et de Recherches Biologiques, 75020 Paris (FR)
(72) Inventeur: SCHERNINSKI, François, F-75011 Paris (FR); QUENTEL, Gabriel, F-75007 Paris (FR)
(74) Mandataire: Burbaud, Eric
(86) Numéro de dépôt international: FR9500344
(87) Numéro de publication internationale: WO9525460

(56) Documents cités:
- EP-A- 0 195 375
- EP-A- 0 512 965
- EP-A- 0 554 643
- US-A- 4 768 513
- US-A- 4 919 114
- US-A- 5 215 095
- US-A- 5 279 298

## Description

L'invention est relative aux dispositifs d'imagerie endoscopique ou fibroscopique en fluorescence dans l'infrarouge.

Plus particulièrement, la présente invention concerne un dispositif d'angiographie destiné à inspecter une paroi d'une cavité corporelle d'un patient après injection interne à ce patient d'un colorant fluorescent pharmaceutiquement acceptable, ce colorant pouvant émettre une lumière dans des longueurs d'onde dites de fluorescence lorsqu'il est excité par une lumière dans des longueurs d'onde dites d'excitation, ce dispositif comportant:
- un conduit flexible ayant une faible section et présentant une extrémité libre qui est destinée à être introduite dans ladite cavité corporelle pour en inspecter la paroi,
- des moyens d'excitation pour émettre à cette extrémité libre une lumière dite d'excitation, dans une première plage de longueur d'onde qui inclut au moins une partie des longueurs d'onde d'excitation du colorant,
- des moyens de réception pour recevoir à ladite extrémité libre une image de la paroi observée, dans une deuxième plage de longueur d'onde qui inclut au moins une partie des longueurs d'onde de fluorescence du colorant et qui ne présente sensiblement aucun recouvrement avec la première plage de longueur d'onde, et
- des moyens de visualisation de l'image reçue.

Un tel dispositif a été divulgué notamment par T. Satoh et al. ("Use of fluorescent electronic endoscopy in evaluation of peptic ulcers", Endoscopy 1991; 23: 313-316) puis par U.K. Franzeck et al. ("Dynamic fluorescence video-endoscopy for intravital evaluation of gastrointestinal mucosal blood flow", Gastrointest. Endosc. 1993; 39; 6: 806-809).

Lorsqu'on utilise ce type de dispositif, on injecte à un patient, par voie intraveineuse, un colorant fluorescent. Sous l'effet de la lumière d'excitation, ce colorant émet une lumière fluorescée qui permet de visualiser le réseau vasculaire dans la paroi de la cavité corporelle examinée. Comme la grande majorité des lésions viscérales s'accompagne de notables modifications vasculaires, on peut ainsi repérer ces lésions.

Les dispositifs connus précités sont conçus pour utiliser comme colorant la fluorescéine, qui présente un très bon rendement en fluorescence. Du fait que la fluorescéine est excitée par la lumière dans le domaine visible et fluoresce également dans le domaine visible, les première et deuxième plages de longueur d'onde correspondant aux dispositifs connus précités sont toutes les deux situées dans le spectre de la lumière visible.

Du fait que la lumière visible est largement absorbée par les tissus formant la paroi de la cavité corporelle à observer, ces dispositifs connus ne permettent donc d'observer que le réseau vasculaire superficiel, lequel est peu intéressant sur le plan clinique.

De plus, la fluorescéine présente également l'inconvénient de diffuser dans le tissu interstitiel formant la paroi à observer. Il en résulte un fond de fluorescence qui brouille l'image.

Il est donc souhaitable d'éviter d'utiliser la fluorescéine comme colorant et, plus généralement, d'éviter d'utiliser un colorant dont les longueurs d'onde d'excitation et de fluorescence sont situées dans le domaine visible.

Par ailleurs, G. Panzardi et al. (EP-A- 0 554 643 et "Choroidal angiography with indocyanine green dye: absorption and fluorescence techniques" European Journal of Ophthalmology/volume 2/no. 2, 1992, pages 83-85) ont divulgué un dispositif d'angiographie permettant d'examiner le réseau vasculaire de la choroïde d'un patient, en utilisant comme colorant le Vert d'Indocyanine, qui présente l'avantage d'une part de permettre de travailler dans le domaine infrarouge, les rayons infrarouges pouvant pénétrer relativement profondément dans les tissus, et d'autre part de ne pas diffuser dans les tissus en dehors du réseau vasculaire. De plus, le Vert d'Indocyanine est particulièrement bien toléré par l'organisme.

Toutefois, le rendement en fluorescence du Vert d'Indocyanine est très faible, de sorte que la lumière fluorescée par la paroi observée est également très faible.

Par conséquent, si le dispositif divulgué par G. Panzardi et al. convient pour une observation directe telle qu'une observation de la choroïde, il serait inutilisable pour observer en fluorescence la paroi d'une cavité corporelle au travers d'un fibroscope ou d'un endoscope, dans la mesure où le fibroscope provoque une atténuation lumineuse importante entre ses extrémités d'entrée et de sortie, l'endoscope provoque une atténuation importante de la lumière d'excitation et une caméra analogique miniaturisée disposée à l'extrémité libre de l'endoscope, du fait de sa miniaturisation, risquerait de générer un bruit de fond important qui perturberait la qualité de l'image et empêcherait un traitement ultérieur de cette image.

La présente invention a donc pour but de proposer un dispositif d'angiographie endoscopique ou fibroscopique en fluorescence qui permette de travailler dans le domaine infrarouge, en utilisant notamment comme colorant le Vert d'Indocyanine ou tout autre colorant pharmaceutiquement acceptable fluorescent dans le domaine infrarouge, et qui permette d'obtenir des images exploitables même si le colorant utilisé présente un mauvais rendement en fluorescence.

A cet effet, selon l'invention, un dispositif du genre en question est essentiellement tel que décrit dans la revendication 1.

Comme la lumière d'excitation et la lumière fluorescée appartiennent au domaine infrarouge, le dispositif selon l'invention permet de visualiser en profondeur le réseau vasculaire de la paroi observée.

D'autre part, l'image captée en fluorescence présente un excellent rapport signal/bruit grâce au fait que la deuxième plage de longueur d'onde ne présente sensiblement pas de recouvrement avec la première plage de longueur d'onde, au contraire du procédé d'angiographie endoscopique par absorption en infrarouge, divulgué notamment par Hidetoshi Ohta et al. en 1991 ("The near infrared electronic endoscope for diagnosis of easophageal varices", Gastrointest. Endosc., 1992; 38: 330-335).

Du fait que les signaux lumineux sont transformés d'emblée en signaux numériques, ce rapport signal/bruit est conservé, ce qui permet un traitement efficace de l'image de façon à en améliorer la visibilité.

Dans des modes de réalisation préférés de l'invention, on a recours à l'une et/ou à l'autre des dispositions suivantes:
- la première plage de longueur d'onde est au moins en partie comprise entre 766 et 815 nm et la deuxième plage de longueur d'onde est au moins en partie comprise entre 825 et 840 nm;
- les moyens d'excitation comportent une source de lumière polychromatique de forte puissance, des moyens pour interrompre séquentiellement l'émission de la source lumineuse, une fibre optique dite d'excitation pour conduire jusqu'à l'extrémité libre du conduit flexible la lumière émise par la source, et un filtre d'excitation disposé pour recevoir la totalité de la lumière qui traverse également la fibre optique d'excitation, ce filtre d'excitation laissant passer sensiblement toute la lumière dans la première plage de longueur d'onde et absorbant sensiblement toute la lumière dans la deuxième plage de longueur d'onde;
- les moyens pour interrompre séquentiellement l'émission de la source lumineuse comportent un cache disposé en amont du filtre d'excitation;
- la source lumineuse est un flash;
- la source lumineuse a une puissance réglable;
- le filtre d'excitation est amovible;
- le dispositif comporte trois filtres couleur ayant chacun une bande passante distincte dans le domaine visible, et des moyens pour interposer sélectivement ou successivement chacun de ces filtres couleur entre la source de lumière et la fibre optique d'excitation;
- les moyens d'excitation comportent une source laser monochromatique émettant une lumière d'excitation infrarouge et une fibre optique dite d'excitation, pour conduire la lumière émise par la source laser jusqu'à l'extrémité libre du conduit flexible;
- les moyens d'excitation envoient un faisceau fin de lumière d'excitation sur la paroi à observer, et comportent des moyens de balayage pour déplacer le faisceau de lumière d'excitation en lui faisant balayer la paroi à observer;
- les moyens de balayage comportent un miroir mobile en rotation autour de deux axes perpendiculaires l'un à l'autre et des moyens de commande de la rotation du miroir sur ces deux axes, le faisceau de lumière d'excitation arrivant sur ce miroir et étant renvoyé par ce miroir vers la paroi à observer;
- le faisceau de lumière d'excitation traverse un miroir fixe semi-réfléchissant avant d'arriver sur le miroir mobile, et ce miroir fixe renvoie vers une cellule détectrice numérique sensible dans l'infrarouge toute lumière qui provient de la paroi à observer dans une direction empruntée par le faisceau de lumière d'excitation après sa réflexion sur le miroir mobile, un filtre d'arrêt étant interposé entre le miroir fixe et la cellule détectrice, ce filtre d'arrêt absorbant sensiblement toute la lumière dans la première plage de longueur d'onde et laissant passer sensiblement toute la lumière dans la deuxième plage de longueur d'onde;
- les moyens de réception comportent une caméra numérique sensible dans l'infrarouge qui est reliée à l'extrémité libre du conduit flexible par une fibre optique de réception, cette fibre optique de réception conduisant jusqu'à la caméra numérique la lumière qu'elle reçoit à l'extrémité libre du conduit flexible, un filtre d'arrêt étant disposé pour être traversé par la lumière conduite par la fibre optique de réception, ce filtre d'arrêt absorbant sensiblement toute la lumière dans la première plage de longueur d'onde et laissant passer sensiblement toute la lumière dans la deuxième plage de longueur d'onde;
- les moyens de réception comprennent une caméra numérique sensible dans l'infrarouge qui est disposée à l'intérieur du conduit flexible, au voisinage de l'extrémité libre de celui-ci, un filtre d'arrêt étant prévu à ladite extrémité libre pour être traversé par tout le flux lumineux reçu par la caméra numérique, ce filtre d'arrêt absorbant sensiblement toute la lumière dans la première plage de longueur d'onde et laissant passer sensiblement toute la lumière dans la deuxième plage de longueur d'onde.

D'autres caractéristiques et avantages de l'invention apparaîtront au cours de la description détaillée suivante de quatre de ses formes de réalisation, données à titre d'exemples non limitatifs, en regard des dessins joints.

Sur les dessins:
- la figure 1 est une vue schématique illustrant une première forme de réalisation de l'invention,
- la figure 2 représente la courbe de transmission spectrale des filtres d'excitation et d'arrêt du dispositif de la figure 1, ainsi que le spectre d'absorption et de fluorescence du Vert d'Indocyanine,
- la figure 3 est une vue schématique partielle d'une deuxième forme de réalisation de l'invention,
- la figure 4 est une vue schématique de l'extrémité libre d'un endoscope dans une troisième forme de réalisation de l'invention, et
- la figure 5 est une vue schématique de l'extrémité libre d'un endoscope dans une quatrième forme de réalisation de l'invention.

Sur les différentes figures, les mêmes références désignent des éléments similaires.

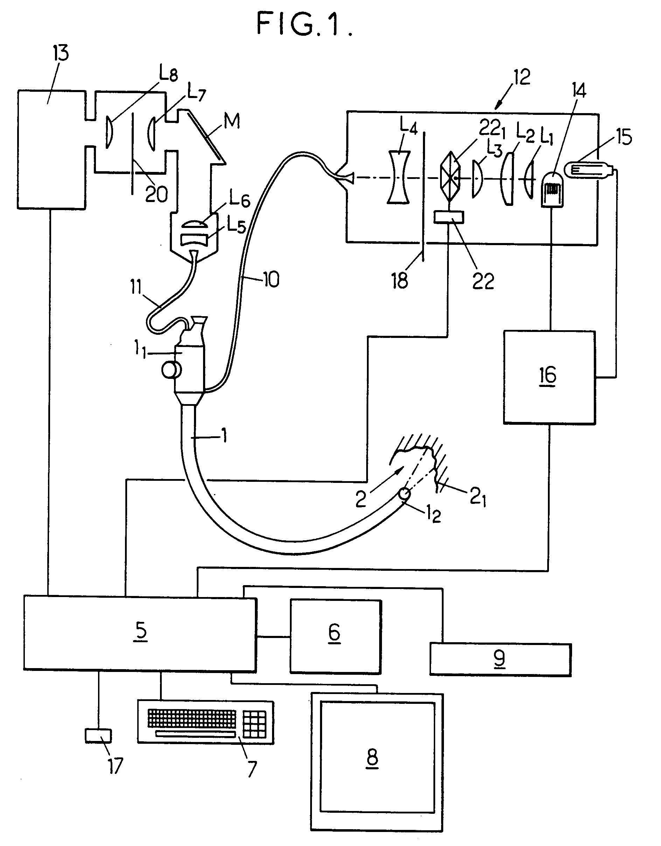

Dans les quatre formes de réalisation de l'invention décrites ci-dessous, le dispositif d'angiographie comporte un conduit flexible 1 pouvant être un fibroscope ou un endoscope, qui s'étend longitudinalement entre une extrémité 1₁ et une extrémité libre 1₂ destinée à être introduite dans une cavité corporelle 2 chez un patient pour en inspecter la paroi 2₁ (voir figure 1).

Le dispositif selon l'invention est destiné à être utilisé après avoir injecté au patient par voie intraveineuse un colorant fluorescent qui peut être excité par un rayonnement infrarouge pour émettre à son tour un rayonnement fluorescent également dans le domaine infrarouge, ce qui permet de visualiser le réseau vasculaire de la paroi observée sur une profondeur relativement importante à partir de l'intérieur de la cavité corporelle, d'où une bonne efficacité dans la détection des lésions entraînant des modifications vasculaires même minimes.

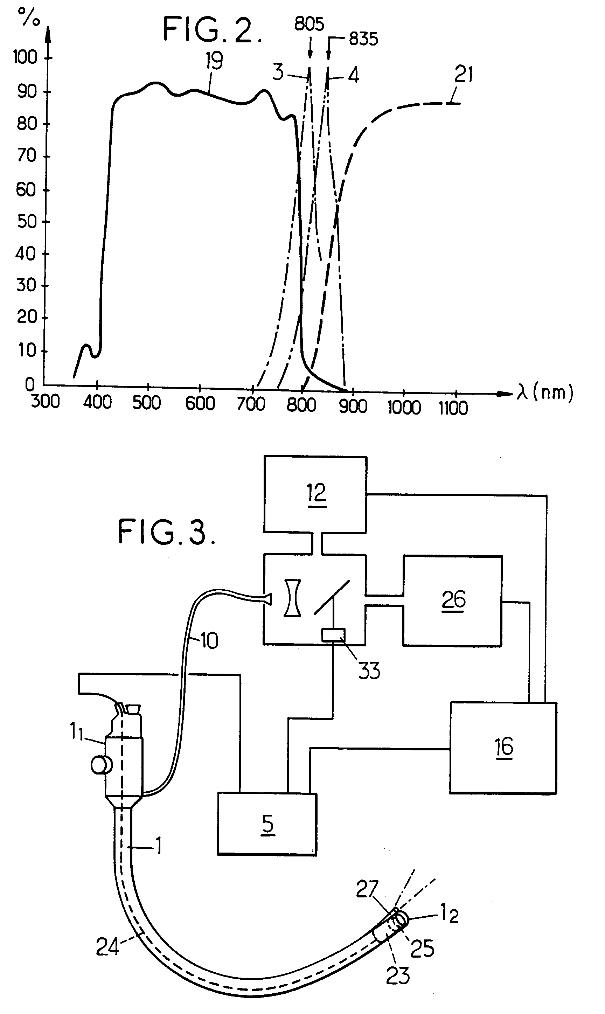

En particulier, dans les formes de réalisation décrites ci-dessous, le dispositif selon l'invention est spécialement conçu pour permettre d'utiliser comme colorant le Vert d'Indocyanine, dont les spectres d'absorption et de fluorescence sont représentés respectivement par les courbes 3 et 4 sur la figure 2.

Comme on peut le voir sur la figure 2, le spectre d'absorption du Vert d'Indocyanine présente un pic pour une longueur d'onde du rayonnement reçu de 805 nm, tandis que le spectre de fluorescence présente un pic pour une longueur d'onde émise de 835 nm.

Le Vert d'Indocyanine présent dans le réseau vasculaire de la paroi 2₁ est excité par une lumière d'excitation émise à l'extrémité 1₂ du fibroscope ou de l'endoscope, dans une première plage de longueur d'onde incluant une partie du spectre infrarouge. De préférence, au moins une partie de la lumière d'excitation doit être émise avec des longueurs d'onde comprises entre 766 et 815 nm, de façon à exciter efficacement le Vert d'Indocyanine.

Cette lumière d'excitation provoque l'émission d'un rayonnement infrarouge par le Vert d'Indocyanine, selon un phénomène de fluorescence, le maximum de rayonnement infrarouge étant émis à une longueur d'onde de 835 nm.

Cette émission de rayonnement infrarouge par fluorescence compose une image de la paroi 2₁ en fluorescence qui est reçue à l'extrémité libre 1₂ du fibroscope ou de l'endoscope. Les signaux lumineux qui composent cette image sont ensuite directement transformés en signaux numériques, généralement électriques: ainsi, on évite de détériorer par un quelconque traitement analogique le rapport signal/bruit des signaux lumineux captés. De cette façon, on peut tirer partie du bon rapport signal/bruit des signaux lumineux composant l'image de la paroi 2₁ en fluorescence, et ce malgré la faible luminosité et le faible contraste de cette image.

Dans les quatre exemples de réalisation qui vont être décrits ci-dessous, les signaux numériques provenant du capteur sont transmis à un micro-ordinateur 5 (voir figure 1) qui est classiquement relié à une unité de stockage de masse 6 telle qu'un disque optique, un clavier 7, un écran haute définition 8 et une imprimante 9.

Le micro-ordinateur 5 comporte un logiciel de traitement numérique de l'image pour augmenter la définition, le contraste et la luminosité de l'image captée.

Ce logiciel peut notamment:
- amplifier les signaux reçus,
- modifier l'histogramme de répartition des niveaux de gris de l'image, de façon à en améliorer la définition et le contraste,
- transformer les niveaux de gris de l'image en niveaux de couleurs, et
- superposer les images.

Grâce à ses performances dans le traitement de l'image, le dispositif selon l'invention permet d'exploiter des images en fluorescence obtenues en utilisant comme colorant le Vert d'Indocyanine, et ce malgré le faible rendement en fluorescence de ce colorant et malgré les atténuations des signaux lumineux qui se produisent dans l'endoscope ou le fibroscope. De plus, grâce à ces performances, le dispositif selon l'invention permet d'utiliser de faibles quantités de colorant.

Le dispositif selon l'invention, utilisé avec le Vert d'Indocyanine, permet l'objectivation de modifications vasculaires, de structures néovasculaires et de tumeurs muqueuses, sous-muqueuses ou parenchymateuses (laparoscopie), à leur stade précoce.

Le dispositif de l'invention permet de repérer des lésions dès les premières minutes suivant l'injection de Vert d'Indocyanine, et au plus tard généralement de 30 à 40 minutes après l'injection de ce colorant.

Dans la première forme de réalisation de l'invention, représentée sur la figure 1, le conduit flexible 1 est un fibroscope, qui contient notamment deux fibres optiques 10, 11 transparentes dans l'infrarouge, savoir une fibre optique 10 dite d'excitation, qui conduit jusqu'à l'extrémité libre 1₂ du fibroscope la lumière d'excitation produite par une source lumineuse 12, et une fibre optique 11 dite de réception, qui conduit vers une caméra numérique 13 l'image de la paroi observée 2₁ telle que cette image est reçue à l'extrémité libre 1₂ du fibroscope.

La source lumineuse 12 comporte d'une part une lampe halogène 14 de 300 watts ou plus, et d'autre part un flash 15 au xénon ayant par exemple une énergie de 300 watt.s, tous deux alimentés par une alimentation 16 à variateur qui est elle-même commandée par le micro-ordinateur 5. Une gâchette 17, reliée au micro-ordinateur 5, permet de déclencher les émissions lumineuses du flash 15 et une prise de vues simultanée par mémorisation de l'image au moment de l'émission lumineuse du flash.

La lumière émise par la lampe halogène 14 ou le flash 15 est transmise à l'entrée de la fibre optique 10 dite d'excitation par l'intermédiaire de diverses lentilles schématiquement montrées en L₁, L₂, L₃ et L₄, réalisées en verre transparent aux rayons infrarouges, de préférence un verre riche en plomb et pauvre en silice et de structure monocubique, et par l'intermédiaire d'un filtre d'excitation 18 dont un exemple de courbe de transmission spectrale est référencé 19 sur la figure 2.

Comme on peut le voir sur la figure 2, le filtre d'excitation 18 laisse sensiblement passer la lumière dans une plage de longueur d'onde comprise entre 400 et 805 nm, et il absorbe sensiblement toute la lumière au-delà de 805 nm. Ainsi, comme on peut le voir en comparant les courbes 3 et 19 de la figure 2, le filtre d'excitation 18 laisse passer suffisamment de lumière infrarouge dans les longueurs d'onde d'excitation du Vert d'Indocyanine, pour provoquer une fluorescence suffisante de ce colorant.

La sortie de la fibre optique 11 de réception est reliée à la caméra numérique 13 par l'intermédiaire d'une optique (comprenant des lentilles schématiquement représentées en L₅, L₆, L₇ et L₈ et un miroir M) également transparente aux rayons infrarouges, et d'un filtre d'arrêt 20 qui arrête sensiblement toute la lumière dans la première plage de longueur d'onde susmentionnée, et qui laisse passer sensiblement toute la lumière dans une deuxième plage de longueur d'onde correspondant aux longueurs d'onde de fluorescence du Vert d'Indocyanine, cette deuxième plage de longueur d'onde ne se recouvrant pas ou sensiblement pas avec la première plage de longueur d'onde.

Un exemple de courbe de transmission spectrale du filtre d'arrêt 20 est représenté sur la figure 2 sous la référence 21. On peut constater, par comparaison avec la courbe de transmission spectrale 19, que le filtre d'arrêt 20 ne laisse passer sensiblement aucune lumière transmise par le filtre d'excitation 18. De plus, en comparant la courbe de transmission spectrale 21 avec le spectre de fluorescence 4 du Vert d'Indocyanine, on peut constater que le filtre d'arrêt 20 laisse passer une partie importante de l'énergie lumineuse émise par fluorescence par le Vert d'Indocyanine, le filtre d'arrêt 20 laissant passer sensiblement toute la lumière au-delà de 825 nm.

Ainsi, l'image reçue par la caméra numérique 13 est exclusivement une image en fluorescence, qui n'est pas brouillée par la lumière d'excitation.

La caméra numérique 13 est une caméra à haute résolution avec une bonne sensibilité dans l'infrarouge, par exemple la caméra commercialisée sous la marque "VIDEK MEGA PLUS" par la Société KODAK.

Avantageusement, les filtres 18 et 20 peuvent être amovibles, de façon à pouvoir utiliser le dispositif pour prendre des images de la paroi 2₁ dans le domaine visible.

Eventuellement, un seul des deux filtres 18 et 20 pourrait être amovible, le filtre fixe étant transparent en lumière visible. La source lumineuse comporte en outre un dispositif d'occultation 22, disposé entre le filtre d'excitation 18 et la lampe 14 et commandé par le micro-ordinateur 5, pour interrompre périodiquement l'émission de la lumière d'excitation, de façon à ne pas endommager le filtre d'excitation 18, le fibroscope ou la paroi 2₁ lorsque la lampe halogène 14 est utilisée à forte puissance, notamment lors d'un examen prolongé. Le système d'occultation 22 peut notamment comprendre un disque 22₁ entraîné en rotation, qui présente des parties transparentes et des parties opaques disposées successivement devant l'entrée de la fibre optique 10. Lorsque la lumière d'excitation est périodiquement interrompue, le logiciel du micro-ordinateur 5 synchronise de préférence la prise de vue avec les périodes d'illumination.

Eventuellement, le disque 22₁ peut être remplacé par un disque présentant une succession de filtres de couleur, par exemple bleu, jaune, rouge. Ainsi, en travaillant non plus en fluorescence mais en lumière visible, il est possible de faire prendre à la caméra 13 une succession de vues monochromes qui peuvent être combinées par le logiciel du micro-ordinateur 5 par superposition, pour reconstituer une image en couleur de la paroi 2₁.

Dans la deuxième forme de réalisation de l'invention, représentée sur la figure 3, le conduit flexible 1 est un endoscope qui présente au voisinage de son extrémité libre 1₂ une caméra numérique 23 miniaturisée, sensible aux rayons infrarouges, un filtre d'arrêt 25, qui laisse passer sensiblement toute la lumière en deça de 750 nm (domaine visible) et au-delà de 830 nm et qui absorbe sensiblement toute la lumière en dehors de ces plages, étant placé devant l'objectif de la caméra numérique 23. La caméra 23 est reliée au micro-ordinateur 5 par une liaison 24.

Dans cette forme de réalisation, la lumière d'excitation peut être produite par une source lumineuse 12 telle que celle déjà décrite ci-dessus; ou bien il est également possible que cette source lumineuse 12 ne comporte pas de filtre d'excitation 18 et n'est utilisée que pour la visualisation de la paroi observée en lumière visible, une diode laser 26 monochromatique étant utilisée pour produire la lumière d'excitation.

Cette diode laser émet alors à une longueur d'onde proche de 805 nm, par exemple 793 ou 815 nm, avec une faible largeur de bande passante, de façon à ne pas émettre au-dessus de 825 nm. Dans ce cas, de préférence, à l'extrémité libre 1₂ de l'endoscope est prévue une optique 27 raccordée à l'extrémité de la fibre optique 10 pour diffuser la lumière monochromatique vers la paroi à observer avec un angle d'observation suffisant, par exemple de 40°.

Un dispositif à miroir pivotant 33, commandé par le micro-ordinateur 5, permet de sélectionner soit la source 12 en lumière visible, soit la diode laser 26 en infrarouge.

De préférence, on peut prévoir un système d'interruption séquentielle de la diode laser 26, similaire au dispositif 22 de la figure 1, cette variante n'étant pas représentée.

On notera que le dispositif de la figure 3 pourrait comporter, à la place du filtre 25 et de la caméra numérique 23 situés à l'intérieur de l'endoscope, une caméra numérique 13 et un filtre 20 disposés à l'extérieur du conduit et reliés à l'extrémité libre de ce conduit par une fibre optique de réception 11, comme décrit ci-dessus en regard de la figure 1 (le conduit étant alors à nouveau un fibroscope).

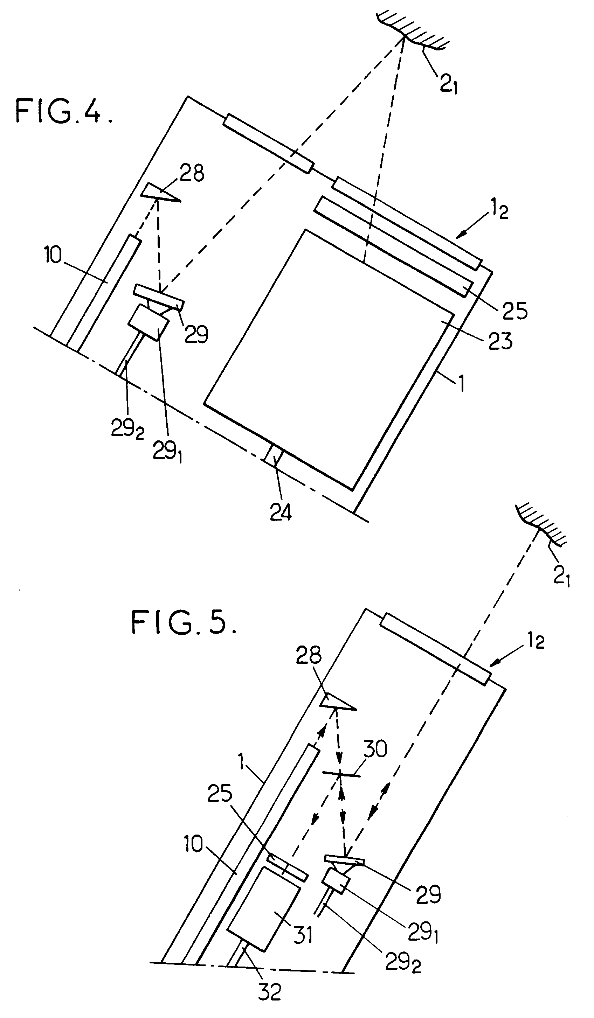

Dans la forme de réalisation représentée sur la figure 4, le conduit flexible 1 est toujours un endoscope et, comme dans l'exemple de la figure 3, la source de lumière d'excitation est une diode laser monochromatique qui est reliée à l'extrémité libre 1₂ de l'endoscope par une fibre optique 10.

A la différence de l'exemple de la figure 3, l'extrémité libre de l'endoscope ne comporte pas d'optique de diffusion 27, mais un miroir fixe 28 qui renvoie le rayon laser vers un miroir mobile 29, lequel miroir mobile renvoie le rayon laser vers la paroi 2₁ à observer.

Le miroir mobile 29 est monté pivotant autour de deux axes perpendiculaires l'un à l'autre, et il est commandé en rotation autour de ces deux axes par un dispositif 29₁ relié au micro-ordinateur 5 au moyen d'une liaison 29₂, de façon que le rayon laser balaie très rapidement la paroi 2₁ à observer.

La lumière émise par fluorescence par le Vert d'Indocyanine circulant dans le réseau vasculaire de la paroi 2₁ est ensuite captée par une caméra numérique 23 au travers d'un filtre d'arrêt 25. La caméra numérique 23 et le filtre d'arrêt 25 sont identiques ou similaires à ceux décrits dans l'exemple de la figure 3, et comme dans cet exemple, ils sont disposés à l'intérieur de l'endoscope 1 au voisinage de son extrémité libre, la caméra numérique 23 étant reliée au micro-ordinateur 5 par une ligne 24.

Dans cette forme de réalisation, la caméra numérique 23 reçoit donc l'image point par point, au fur et à mesure du balayage de la paroi 2₁ par le rayon laser, et le logiciel contenu dans le micro-ordinateur 5 reconstitue ensuite l'image de la paroi 2₁ au bout d'un cycle complet de balayage par le rayon laser.

Du fait que l'énergie lumineuse du rayon laser est concentrée en un point de la paroi 2₁ à un instant donné, la réponse du Vert d'Indocyanine en fluorescence est intense, de sorte que l'image ainsi obtenue est de grande qualité, sans pour autant que les tissus de la paroi 2₁ risquent d'être abîmés par le rayon laser, puisque celui-ci ne fait que balayer la paroi.

Le dispositif représenté sur la figure 5 fonctionne de façon similaire à celui de la figure 4, mais s'en différencie en ce qu'il ne comporte pas une caméra numérique 23, mais une cellule numérique photosensible 31, sensible dans l'infrarouge, reliée au micro-ordinateur par une ligne 32.

Un miroir fixe 30 semi-réfléchissant est interposé entre le miroir fixe 28 et le miroir mobile 29, de façon à laisser passer le rayon laser du miroir 28 vers le miroir 29, mais à renvoyer vers la cellule numérique 31 la lumière provenant de la paroi 2₁ et réfléchie par le miroir 29, le filtre d'arrêt 25, identique ou similaire au filtre 25 de la figure 4, étant interposé entre le miroir 30 semi-réfléchissant et la cellule numérique 31.

## Revendications

1. Dispositif d'angiographie destiné à inspecter une paroi (2₁) d'une cavité corporelle (2) d'un patient après injection intraveineuse à ce patient d'un colorant fluorescent pharmaceutiquement acceptable, ce colorant pouvant émettre une lumière dans des longueurs d'onde dites de fluorescence lorsqu'il est excité par une lumière dans des longueurs d'onde dites d'excitation, ce dispositif comportant:
- un conduit (1) présentant une extrémité libre (1₂),
- des moyens d'excitation (12, 10, 26, 28, 29) pour émettre à cette extrémité libre une lumière dite d'excitation, dans une première plage de longueur d'onde qui inclut au moins une partie des longueurs d'onde d'excitation du colorant,
- des moyens de réception (5, 11, 20, 13, 23, 25, 31) pour recevoir à ladite extrémité libre une image de la paroi observée, dans une deuxième plage de longueur d'onde qui inclut au moins une partie des longueurs d'onde de fluorescence du colorant et qui ne présente sensiblement aucun recouvrement avec la première plage de longueur d'onde,
- chacune desdites première et deuxième plages de longeur d'onde appartient au moins en partie au domaine infrarouge, et
- des moyens de visualisation (8, 9) de l'image reçue,
ce dispositif étant caractérisé en ce que le conduit (1) est flexible et a une faible section, son extrémité libre étant destinée à être introduite dans une cavité corporelle pour en inspecter la paroi, et en ce que les moyens de réception comportent un capteur numérique (13, 23, 31) pour transformer directement en signaux numériques l'image captée, et des moyens de traitement numérique (5) pour augmenter la définition, le contraste et la luminosité de l'image captée.

2. Dispositif selon la revendication 1, dans lequel la première plage de longueur d'onde est au moins en partie comprise entre 766 et 815 nm et dans lequel la deuxième plage de longueur d'onde est au moins en partie comprise entre 825 et 840 nm.

3. Dispositif selon l'une quelconque des revendications 1 et 2, dans lequel les moyens d'excitation comportent une source de lumière polychromatique de forte puissance (14, 15), des moyens (22, 16) pour interrompre séquentiellement l'émission de la source lumineuse, une fibre optique (10) dite d'excitation pour conduire jusqu'à l'extrémité libre (1₂) du conduit flexible la lumière émise par la source, et un filtre d'excitation (18) disposé pour recevoir la totalité de la lumière qui traverse également la fibre optique d'excitation, ce filtre d'excitation laissant passer sensiblement toute la lumière dans la première plage de longueur d'onde et absorbant sensiblement toute la lumière dans la deuxième plage de longueur d'onde.

4. Dispositif selon la revendication 3, dans lequel les moyens pour interrompre séquentiellement l'émission de la source lumineuse comportent un cache (22₁) disposé en amont du filtre d'excitation (18).

5. Dispositif selon la revendicaiton 3, dans lequel la source lumineuse est un flash (15).

6. Dispositif selon l'une quelconque des revendications 3 à 5, dans lequel la source lumineuse a une puissance réglable.

7. Dispositif selon l'une quelconque des revendications 3 à 6, dans lequel le filtre d'excitation est amovible.

8. Dispositif selon l'une quelconque des revendications 3 à 7, comportant trois filtres couleur ayant chacun une bande passante distincte dans le domaine visible, et des moyens (22) pour interposer sélectivement ou successivement chacun de ces filtres couleur entre la source de lumière (14, 15) et la fibre optique d'excitation (10).

9. Dispositif selon l'une quelconque des revendications 1 et 2, dans lequel les moyens d'excitation comportent une source laser monochromatique (26) émettant une lumière d'excitation infrarouge et une fibre optique (10) dite d'excitation, pour conduire la lumière émise par la source laser jusqu'à l'extrémité libre (1₂) du conduit flexible.

10. Dispositif selon l'une quelconque des revendications précédentes, dans lequel les moyens d'excitation (26) envoient un faisceau fin de lumière d'excitation sur la paroi (2₁) à observer, et comportent des moyens de balayage (29) pour déplacer le faisceau de lumière d'excitation en lui faisant balayer la paroi à observer.

11. Dispositif selon la revendication 10, dans lequel les moyens de balayage comportent un miroir (29) mobile en rotation autour de deux axes perpendiculaires l'un à l'autre et des moyens de commande (29₁) de la rotation du miroir sur ces deux axes, le faisceau de lumière d'excitation arrivant sur ce miroir et étant renvoyé par ce miroir vers la paroi à observer.

12. Dispositif selon la revendication 11, dans lequel le faisceau de lumière d'excitation traverse un miroir fixe semi-réfléchissant (30) avant d'arriver sur le miroir mobile (29), et ce miroir fixe (30) renvoie vers une cellule détectrice numérique (31) sensible dans l'infrarouge toute lumière qui provient de la paroi à observer (2₁) dans une direction empruntée par le faisceau de lumière d'excitation après sa réflexion sur le miroir mobile, un filtre d'arrêt (25) étant interposé entre le miroir fixe et la cellule détectrice, ce filtre d'arrêt absorbant sensiblement toute la lumière dans la première plage de longueur d'onde et laissant passer sensiblement toute la lumière dans la deuxième plage de longueur d'onde.

13. Dispositif selon l'une quelconque des revendications 1 à 11, dans lequel les moyens de réception comportent une caméra numérique (13) sensible dans l'infrarouge qui est reliée à l'extrémité libre (1₂) du conduit flexible par une fibre optique de réception (11), cette fibre optique de réception conduisant jusqu'à la caméra numérique la lumière qu'elle reçoit à l'extrémité libre du conduit flexible, un filtre d'arrêt (20) étant disposé pour être traversé par la lumière conduite par la fibre optique de réception, ce filtre d'arrêt absorbant sensiblement toute la lumière dans la première plage de longueur d'onde et laissant passer sensiblement toute la lumière dans la deuxième plage de longueur d'onde.

14. Dispositif selon l'une quelconque des revendications 1 à 11, dans lequel les moyens de réception comprennent une caméra numérique (23) sensible dans l'infrarouge, qui est disposée à l'intérieur du conduit flexible (1), au voisinage de l'extrémité libre (1₂) de celui-ci, un filtre d'arrêt (25) étant prévu à ladite extrémité libre pour être traversé par tout le flux lumineux reçu par la caméra numérique, ce filtre d'arrêt absorbant sensiblement toute la lumière dans la première plage de longueur d'onde et laissant passer sensiblement toute la lumière dans la deuxième plage de longueur d'onde.

## Claims

1. An angiography device for inspecting a wall (2₁) of a body cavity (2) of a patient after the patient has been intravenously injected with a pharmaceutically acceptable fluorescent dye, the dye being capable of emitting light at "fluorescence" wavelengths when excited by light at "excitation" wavelengths, the device comprising:
. a duct (1) having a free end (1₂);
. excitation means (12, 10, 26, 28, 29) for emitting "excitation" light from said free end in a first range of wavelengths including at least a portion of the excitation wavelengths of the dye;
. reception means (5, 11, 20, 13, 23, 25, 31) for receiving, at said free end, an image of the observed wall in a second wavelength range which includes at least a portion of the fluorescence wavelengths of the dye and which has substantially no overlap with the first wavelength range;
. each of said first and second wavelength ranges lies at least in part in the infrared range; and
. display means (8, 9) for displaying the received image;
the device being characterized in that the duct (1) is flexible and has a small section, its free end being designed to be inserted in a body cavity to inspect the wall thereof, and in that the reception means include a digital sensor (13, 23, 31) for directly transforming the image as sensed into digital signals, and digital processing means (5) for increasing definition, contrast, and brightness in the image as sensed.

2. A device according to claim 1, in which the first wavelength range lies, at least in part, between 766 nm and 815 nm, and in which the second wavelength range lies, at least in part, between 825 nm and 840 nm.

3. A device according to claim 1 or 2, in which the excitation means include a high power polychromatic light source (14, 15), means (22, 16) for sequentially interrupting emission from the light source, an "excitation" optical fiber (10) for conveying the light emitted by the source to the free end (1₂) of the flexible duct, and an excitation filter (18) disposed to receive all of the light that also passes along the excitation optical fiber, said excitation filter allowing substantially all of the light in the first wavelength range to pass and absorbing substantially all of the light in the second wavelength range.

4. A device according to claim 3, in which the means for sequentially interrupting the emission from the light source comprise a mask (22₁) disposed upstream from the excitation filter (18).

5. A device according to claim 3, in which the light source is a flash lamp (15).

6. A device according to any one of claims 3 to 5, in which the light source is of adjustable power.

7. A device according to any one of claims 3 to 6, in which the excitation filter is removable.

8. A device according to any one of claims 3 to 7, including three color filters each having a separate passband in the visible range, and means (22) for selectively or successively interposing each of said color filters between the light source (14, 15) and the excitation optical fiber (10).

9. A device according to claim 1 or 2, in which the excitation means comprise a monochromatic laser source (26) emitting infrared excitation light, and an "excitation" optical fiber (10) for conveying the light emitted by the laser source to the free end (1₂) of the flexible duct.

10. A device according to any preceding claim, in which the excitation means (26) apply a narrow excitation light beam on the wall (2₁) to be observed, and include scanning means (29) for moving the excitation light beam so as to cause it to scan the wall to be observed.

11. A device according to claim 10, in which the scanning means comprise a mirror (29) pivotable about two mutually perpendicular axes, and control means (29₁) for controlling pivoting of the mirror about both of said axes, the excitation light beam reaching said mirror and being reflected thereby towards the wall to be observed.

12. A device according to claim 11, in which the excitation light beam passes through a fixed semi-reflecting mirror (30) before reaching the moving mirror (29), said fixed mirror (30) reflecting, towards a digital detector cell (31) sensitive in the infrared, all light coming from the wall to be observed (2₁) along a direction travelled by the excitation light beam after it has been reflected on the moving mirror, a stop filter (25) being interposed between the fixed mirror and the detector cell, said stop filter absorbing substantially all light in the first wavelength range and passing substantially all light in the second wavelength range.

13. A device according to any one of claims 1 to 11, in which the reception means comprise a digital camera (13) responsive in the infrared and connected to the free end (1₂) of the flexible duct by a reception optical fiber (11), said reception optical fiber conveying the light it receives at the free end of the flexible duct to the digital camera, a stop filter (20) being disposed so that the light conveyed by the reception optical fiber passes therethrough, said stop filter absorbing substantially all light in the first wavelength range and passing substantially all light in the second wavelength range.

14. A device according to any one of claims 1 to 11, in which the reception means comprise a digital camera (23) that is sensitive in the infrared and that is disposed inside the flexible duct (1) in the vicinity of the free end (1₂) thereof, a stop filter (25) being provided at said free end so that all of the light flux received by the digital camera passes therethrough, said stop filter absorbing substantially all light in the first wavelength range and passing substantially all light in the second wavelength range.

## Patentansprüche

1. Angiographische Vorrichtung zur Untersuchung einer Wand (2₁) eines Körperhohlraums (2) eines Patienten, dem ein pharmazeutisch verträglicher fluoreszierender Farbstoff intravenös injiziert wurde, welcher bei Stimulierung durch Licht mit sogenannten Erregerwellenlängen ein Licht mit sogenannten Fluoreszenz-Wellenlängen aussenden kann, wobei die Vorrichtung die folgenden Bestandteile enthält:
- eine Röhre (1) mit einem freien Ende (1₂),
- Erregermittel (12, 10, 26, 28, 29) zum Aussenden eines sogenannten Erregerlichts am freien Ende, welches in einem Wellenlängenbereich liegt, der wenigstens einen Teil der den Farbstoff stimulierenden Wellenlängen enthält,
- Empfangsmittel (5, 11, 20, 13, 23, 25, 31) zum Empfang von in einem zweiten Wellenlängenbereich liegenden Abbildungssignalen der untersuchten Wand am freien Ende, wobei der zweite Wellenlängenbereich wenigstens einen Teil der Fluoreszenzwellenlängen des Farbstoffs umfaßt und sich im wesentlichen nicht mit dem ersten Wellenlängenbereich deckt,
- wobei sowohl der erste als auch der zweite Wellenlängenbereich wenigstens teilweise im Infrarotbereich liegt, sowie
- Visualisierungsmittel (8, 9) für die empfangenen Abbildungssignale,
gekennzeichnet dadurch, daß die Röhre (1) biegsam ist und einen kleinen Querschnitt aufweist und daß ihr freies Ende zur Untersuchung der Wand eines Körperhohlraums in diesen Körperhohlraum eingeführt werden kann, und dadurch, daß die Empfangsmittel einen Digital-Wandler (13, 23, 31) zum direkten Umwandeln der empfangenen Abbildungssignale in digitale Daten sowie Mittel (5) zur Verarbeitung digitaler Daten zur Erhöhung der Auflösung, des Kontrasts und der Helligkeit der empfangenen Abbildungssignale enthält.

2. Vorrichtung nach Anspruch 1, wobei der erste Wellenlängenbereich wenigstens teilweise zwischen 766 und 815 nm und der zweite Wellenlängenbereich wenigstens teilweise zwischen 825 und 840 nm liegt.

3. Vorrichtung nach Anspruch 1 oder 2, wobei die Erregermittel eine polychromatische Hochleistungs-Lichtquelle (14, 15), Mittel (22, 16) zum sequentiellen Unterbrechen der Ausstrahlung der Lichtquelle, einen sogenannten Erreger-Lichtwellenleiter (10) zum Leiten des von der Quelle ausgestrahlten Lichts bis zum freien Ende (1₂) der biegsamen Röhre und einen Erregerfilter (18) zum Empfang des gesamten, gleichmäßig den Erreger-Lichtwellenleiter durchfließenden Lichts umfaßt, wobei der Erregerfilter im wesentlichen das gesamte im ersten Wellenlängenbereich liegende Licht durchläßt und im wesentlichen das gesamte im zweiten Wellenlängenbereich liegende Licht absorbiert.

4. Vorrichtung nach Anspruch 3, wobei die Mittel zum sequentiellen Unterbrechen der Ausstrahlung der Lichtquelle eine in Strömungsrichtung vor dem Erregerfilter (18) liegende Abdeckung (22₁) umfassen.

5. Vorrichtung nach Anspruch 3, wobei ein Blitzgerät (15) als Lichtquelle dient.

6. Vorrichtung nach einem der Ansprüche 3 bis 5, wobei die Leistung der Lichtquelle regelbar ist.

7. Vorrichtung nach einem der Ansprüche 3 bis 6, wobei der Erregerfilter abnehmbar ist.

8. Vorrichtung nach einem der Ansprüche 3 bis 7, enthaltend drei Farbfilter, die eine jeweilige bestimmte Durchlaßbandbreite im sichtbaren Bereich aufweisen, sowie Mittel (22) zum selektiven oder sukzessiven Einschieben dieser Farbfilter zwischen die Lichtquelle (14, 15) und den Erreger-Lichtwellenleiter (10).

9. Vorrichtung nach Anspruch 1 oder 2, wobei die Erregermittel eine ein infrarotes Erregerlicht ausstrahlende monochromatische Laserquelle (26) sowie einen sogenannten Erreger-Lichtwellenleiter (10) umfaßen, der zum Weiterleiten des von der Laserquelle ausgestrahlten Lichts zum freien Ende (1₂) der biegsamen Röhre dient.

10. Vorrichtung nach einem der vorherigen Ansprüche, wobei die Erregermittel (26) einen dünnen Erregerlichtstrahl auf die zu betrachtende Wand (2₁) aussenden, enthaltend Abtastmittel (29) zum Umlenken des Erregerlichtstrahls in einer Weise, daß dieser die zu betrachtende Wand abtastet.

11. Vorrichtung nach Anspruch 10, wobei die Abtastmittel einen beweglichen Spiegel (29), welcher sich um zwei senkrecht zueinander stehenden Achsen drehen kann, und Steuermittel (29₁) für die Drehbewegung des Spiegels um diese beiden Achsen enthält, wobei der Erregerlichtstrahl auf den Spiegel auftrifft und von diesem Spiegel auf die zu betrachtende Wand umgelenkt wird.

12. Vorrichtung nach Anspruch 11, wobei der Erregerlichtstrahl einen semi-reflektierenden feststehenden Spiegel (30) passiert, ehe er auf den beweglichen Spiegel (29) trifft, wobei dieser feststehende Spiegel (30) das gesamte von der zu betrachtenden Wand (2₁) in einer vom Erregerlichtstrahl nach seiner Reflektion auf dem beweglichen Spiegel eingeschlagenen Richtung eintreffende Licht auf eine im Infrarotbereich empfindliche, digitale Detektorzelle (31) umlenkt, wobei ein Verschlußfilter (25) zwischen dem feststehenden Spiegel und der Detektorzelle angebracht ist und dieser Verschlußfilter im wesentlichen das gesamte im ersten Wellenlängenbereich liegende Licht absorbiert und im wesentlichen das gesamte im zweiten Wellenlängenbereich liegende Licht durchläßt.

13. Vorrichtung nach einem der Ansprüche 1 bis 11, wobei die Empfangsmittel eine im Infrarotbereich empfindliche digitale Kamera (13) umfassen, welche mittels eines Empfänger-Lichtwellenleiters (11) mit dem freien Ende (1₂) der biegsamen Röhre verbunden ist, wobei der Empfänger-Lichtwellenleiter das am freien Ende der biegsamen Röhre von dieser empfangene Licht zur digitalen Kamera leitet, und wobei die Empfangsmittel weiterhin einen Verschlußfilter (20) enthalten, welcher so angeordnet ist, daß ihn das vom Empfangs-Lichtwellenleiter weitergeleitete Licht passiert, und welcher im wesentlichen das gesamte im ersten Wellenlängenbereich liegende Licht absorbiert und im wesentlichen das gesamte im zweiten Wellenlängenbereich liegende Licht durchläßt.

14. Vorrichtung nach einem der Ansprüche 1 bis 11, wobei die Empfangsmittel eine im Infrarotbereich empfindliche digitale Kamera (23), welche im Innern der biegsamen Röhre (1) nahe deren freien Ende (1₂) angeordnet ist, sowie einen Verschlußfilter (25) umfassen, welcher an diesem freien Ende so angeordnet ist, daß er vom Lichtfluß zur digitalen Kamera passiert wird, wobei der Verschlußfilter im wesentlichen das gesamte im ersten Wellenlängenbereich liegende Licht absorbiert und im wesentlichen das gesamte im zweiten Wellenlängenbereich liegende Licht durchläßt.
